# EUROPEAN PATENT APPLICATION

(11) **EP 4 159 438 A1**
(43) Date of publication of application: **05.04.2023**
(21) Application number: 21812083.0
(22) Date of filing: 24.05.2021
(51) Int. Cl.: B32B 27/22, B32B 27/32, C09J 11/06, G01N 37/00, C09J 123/00, B32B 7/023

(54) **JOINED BODY AND METHOD FOR MANUFACTURING SAME**

(30) Priority: 29.05.2020 JP 2020094891
(71) Applicant: Zeon Corporation, Tokyo 100-8246 (JP)
(72) Inventor: CHIBA Daido, Tokyo 100-8246 (JP); KOMATSUBARA Takuya, Tokyo 100-8246 (JP)
(74) Representative: Maiwald GmbH
(86) International application number: PCT/JP2021/019665
(87) International publication number: WO 2021/241517

(57) **Abstract**

The purpose of the present invention is to provide an assemblymethod for manufacturing with which deformation caused by heating during joining is inhibited, peeling apart of the assembly is facilitated, protein adsorption is suppressed, and low haze is achieved. An assembly [3] includes one or more types of shaped products [1] that are joined via a joining layer [2] having a material constitution different from the shaped products; and a method for manufacturing the same. The joining layer [2] contains: 100 parts by weight of a cycloolefin resin [4]; and a softening agent [5]. The content of the softening agent [5] in the joining layer [2] is 6 parts by weight to 99 parts by weight of the softening agent [5] relative to 100 parts by weight of the cycloolefin resin [4]. The joining layer [2] has a haze of 1.0 or less.

## Description

### TECHNICAL FIELD

The present disclosure relates to an assembly and a method for manufacturing the same.

### BACKGROUND

A method of producing an assembly through joining of substrates can be used, for example, in the production of a chip (microchannel chip) having a fine channel or reactor on the order of micrometers formed at a joining surface of the substrates. Such microchannel chips can be used in various fields for analysis and testing of biological substances such as DNA, RNA, and proteins, drug discovery/pharmaceutical development, organic synthesis, water quality analysis, and so forth.

A resin microchannel chip may be produced by arranging a joining layer between a resin substrate having a microchannel formed on at least one surface thereof and a resin lid substrate serving as a lid material and performing heating thereof to cause joining (for example, refer to Patent Literature (PTL) 1 to 4).

### CITATION LIST

### Patent Literature

PTL 1: JP5948248B2
PTL 2: WO2014/178439A1
PTL 3: WO2011/083809A1
PTL 4: JP5948248B2

### SUMMARY

### (Technical Problem)

When an assembly such as a microchannel chip is produced by joining shaped products such as substrates via a joining layer, it is desirable to prevent deformation cause by heating during joining and to facilitate peeling apart of the assembly for recycling, etc., or in order to recover cells or medication after testing (i.e., post-joining reworkability is desirable). It is also desirable to suppress protein adsorption to a channel used for analysis, testing, or the like of a biological substance and to reduce haze and achieve good transparency of the joining layer in order to increase the accuracy of optical signal detection.

Accordingly, an object of the present disclosure is to provide an assembly and method for manufacturing the same with which deformation caused by heating during joining is inhibited and peeling apart of the assembly is facilitated due to joining of shaped products being possible at a comparatively low temperature, and with which protein adsorption is suppressed and haze is reduced.

### (Solution to Problem)

As a result of diligent research aimed at solving the problem set forth above, the inventors discovered that by compounding a softening agent in a joining layer, it is possible to join shaped products to each other at a comparatively low temperature, which inhibits deformation caused by heating during joining and facilitates peeling apart of an assembly, and that by using a specific softening agent, protein adsorption is suppressed and haze is reduced. In this manner, the inventors completed the present disclosure.

Thus, the present disclosure provides the following microchannel chips and method for manufacturing the same.
{1} An assembly [3] comprising at least one or more types of shaped products [1] that are joined via a joining layer [2] having a material constitution different from the shaped products, wherein
   the joining layer [2] contains: 100 parts by weight of a cycloolefin resin [4]; and a softening agent [5],
   content of the softening agent [5] in the joining layer [2] is 6 parts by weight to 99 parts by weight of the softening agent [5] relative to 100 parts by weight of the cycloolefin resin [4], and
   the joining layer [2] has a haze of 1.0 or less.
{2} The assembly [3] according to the foregoing {1}, wherein the cycloolefin resin [4] has a number-average molecular weight of 12,500 or more.
{3} The assembly [3] according to the foregoing {1} or {2}, wherein the cycloolefin resin [4] has a glass-transition temperature of 100°C or lower.
{4} The assembly [3] according to any one of the foregoing {1} to {3}, wherein the softening agent [5] is formed of a hydrogenated product of liquid paraffin.
{5} The assembly [3] according to any one of the foregoing {1} to {4}, wherein the cycloolefin resin [4] has an alkoxysilyl group.
{6} The assembly [3] according to any one of the foregoing {1} to {5}, wherein the shaped products [1] are an organic material, and a surface of the shaped products [1] is subjected to one or more treatments selected from the group consisting of plasma irradiation, ultraviolet irradiation, corona discharge, and flame spraying.

### (Advantageous Effect)

According to the present disclosure, it is possible to provide an assembly and method for manufacturing the same with which deformation caused by heating during joining is inhibited, peeling apart of the assembly is facilitated, protein adsorption is suppressed, and low haze is achieved.

### BRIEF DESCRIPTION OF THE DRAWINGS

In the accompanying drawings:

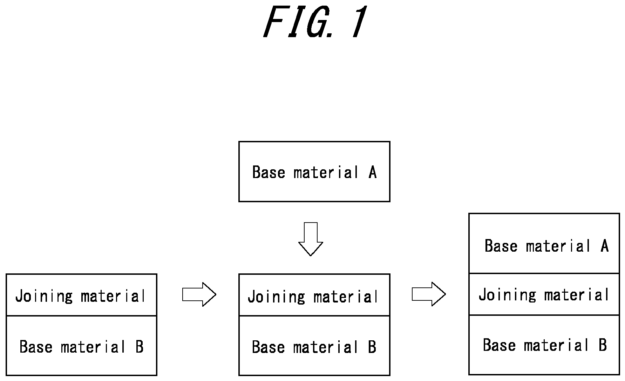

FIG. 1 is a conceptual diagram of a presently disclosed method for manufacturing an assembly; and
FIG. 2 illustrates one example of a presently disclosed assembly.

### DETAILED DESCRIPTION

The following provides a detailed description of embodiments of the present disclosure.

### (Assembly)

A presently disclosed assembly [3] includes one or more types of shaped products [1] that are joined via a joining layer [2] having a material constitution different from the shaped products. A feature of the presently disclosed assembly [3] is that the joining layer [2] has a material constitution in which 6 parts by weight to 99 parts by weight of a softening agent [5] is compounded relative to 100 parts by weight of a cycloolefin resin [4] such that the joining layer [2] has a haze of 1.0 or less.

The presently disclosed assembly [3] may be used for a microchannel chip, a test vessel, or an image display panel, for example.

### <Shaped products>

The shaped products [1] referred to in the present disclosure act as substrates. The shaped products [1] normally have the shape of a flat plate. In the assembly [3], the shaped products [1] are normally a pair (i.e., two), but may be three or more. In a case in which the assembly [3] is to be used as a microchannel chip, a microchannel may be formed on at least one surface of a shaped product [1]. The formation of a microchannel on a shaped product [1] can be achieved by a microprocessing technique such as photolithography or thermal imprinting, cutting, injection molding, or the like, for example.

The shaped products [1] are formed of at least one type of material. In other words, the shaped products [1] may be formed from the same material or may be formed from different materials. The material of the shaped products [1] may be an organic material or an inorganic material. The organic material may be a cycloolefin resin, a polycarbonate-based resin, an aromatic polyether ketone-based resin, a (meth)acrylic-based resin, a vinyl alicyclic hydrocarbon-based resin, or an aromatic vinyl-based resin, for example. Herein, the term "(meth)acrylic" means acrylic and/or methacrylic. The inorganic material may be a metal (for example, an iron alloy such as stainless steel), a metal oxide (glass or ceramic), or the like. In a case in which the assembly [3] is to be used as a microchannel chip, it is preferable to use a cycloolefin resin as a substrate resin, and more preferable to use a cycloolefin resin having a water absorption of 0.01 mass% or less as a substrate resin from a viewpoint of there being little reduction of joint strength over time and reduction of optical stability due to water absorption and of a microchannel chip having excellent durability being obtained.

Note that it is preferable that a norbornene-based monomer is used as a monomer of the cycloolefin resin that may be used as a material of the shaped products [1]. The norbornene-based monomer is a monomer having a norbornene ring. The norbornene-based monomer may be a bicyclic monomer such as bicyclo[2.2.1]hept-2-ene (commonly referred to as norbornene), 5-ethylidene-bicyclo[2.2.1]hept-2-ene (commonly referred to as ethylidene norbornene), or a derivative of either thereof (derivative having a substituent on a ring); a tricyclic monomer such as tricyclo[5.2.1.0^{2,6}]deca-3,8-diene (commonly referred to as dicyclopentadiene) or a derivative thereof; a tetracyclic monomer such as tetracyclo[7.4.0.0^{2,7}.1^{10,13}]tetradeca-2,4,6,11-tetraene (commonly referred to as methanotetrahydrofluorene), tetracyclo[6.2.1.1^{3,6}.0^{2,7}]dodec-4-ene (commonly referred to as tetracyclododecene), 9-ethylidenetetracyclo[6.2.1.1^{3,6}.0^{2,7}]dodec-4-ene, or a derivative of any thereof; or the like, for example. These monomers may have a substituent at any position. The cycloolefin resin that may be used as a material of the shaped products [1] may be an addition polymer, a ring-opened polymer, or a hydrogenated product of either thereof, and is preferably a ring-opened polymer or a hydrogenated ring-opened polymer. The cycloolefin resin may be amorphous or crystalline, but is preferably amorphous.

The aforementioned ring-opened polymer can be produced by a method using a metathesis reaction catalyst (ring-opening polymerization catalyst) such as a ruthenium carbene complex catalyst described in WO2010/110323A1, a method using a ring-opening polymerization catalyst such as a tungsten(phenylimide)tetrachloride tetrahydrofuran complex or tungsten hexachloride described in JP2015-54885A, or the like, for example.

Moreover, the method by which a hydrogenated alicyclic structure-containing ring-opened polymer is produced through hydrogenation of the ring-opened polymer may be a method using a hydrogenation catalyst described in WO2010/110323A1, or the like, for example. For example, a hydrogenated alicyclic structure-containing ring-opened polymer can be produced by using a ruthenium carbene complex catalyst such as described above as a ring-opening polymerization catalyst in order to produce an alicyclic structure-containing polymer and then also using the ruthenium carbene catalyst in that form as a hydrogenation catalyst in order to hydrogenate the alicyclic structure-containing ring-opened polymer.

The number-average molecular weight of the cycloolefin resin that may be used as a material of the shaped products [1] may, for example, be 5,000 or more, preferably 7,500 or more, and more preferably 10,000 or more. Moreover, the number-average molecular weight of the cycloolefin resin may, for example, be 200,000 or less, preferably 100,000 or less, and more preferably 80,000 or less. The molecular weight distribution (Mw/Mn) of the cycloolefin resin may, for example, be 1.2 or more, preferably 1.5 or more, and more preferably 2.0 or more. Moreover, the molecular weight distribution (Mw/Mn) of the cycloolefin resin may, for example, be 6.0 or less, preferably 5.0 or less, and more preferably 4.5 or less.

The molecular weight (number-average molecular weight Mn) can be determined as a standard polyisoprene-equivalent value through measurement by gel permeation chromatography (GPC) as described in the EXAMPLES section of the present specification. For example, the molecular weight may be determined as a standard polyisoprene-equivalent value through measurement by GPC with cyclohexane as an eluent. In a case in which the sample does not dissolve in cyclohexane, the molecular weight may be determined as a standard polystyrene-equivalent value through measurement by GPC with tetrahydrofuran (THF) as an eluent.

The glass-transition temperature Tg of the cycloolefin resin that may be used as a material of the shaped products [1] is preferably 80°C or higher, and more preferably 100°C or higher. Good dimensional stability is obtained through Tg being within a range such as set forth above. Moreover, the glass-transition temperature Tg is preferably 170°C or lower, and more preferably 160°C or lower. Oxidation is inhibited during processing and shaping, and good external appearance with little burning or yellowing is obtained through Tg being within a range such as set forth above.

The glass-transition temperature referred to in the present disclosure can be measured by differential scanning calorimetry (DSC) based on JIS-K7121.

The glass-transition temperature of the cycloolefin resin can be adjusted as appropriate in accordance with the type(s) of monomer(s) used in polymerization, the mixing ratio, the average molecular weight, the molecular weight distribution, and so forth.

In the case of a shaped product [1] that is an organic material, a surface of the shaped product [1] (at least a surface at a side that is joined) may be subjected to one or more treatments selected from the group consisting of plasma irradiation, ultraviolet irradiation, corona discharge, and flame spraying.

### <Joining layer>

The joining layer [2] is a member for joining the shaped products [1] and may be formed on at least one of the shaped products [1]. The joining layer [2] has a material constitution in which 6 parts by weight to 99 parts by weight of a softening agent [5] is compounded relative to 100 parts by weight of a cycloolefin resin [4] such that the haze of the joining layer [2] is 1.0 or less. The haze of the joining layer [2] is 1.0 or less, preferably 0.8 or less, and more preferably 0.5 or less. The haze can be measured by a method described in the EXAMPLES section of the present specification (method using a measurement device such as a haze meter (NDH-7000SP produced by Nippon Denshoku Industries Co., Ltd.)), for example.

In a case in which the presently disclosed assembly [3] is to be used as a microchannel chip for analysis or testing of a biological substance such as DNA, RNA, or a protein, the joining layer [2] is preferably formed of a material to which such biological substances do not adsorb.

The cycloolefin resin [4] used for the joining layer [2] may, for example, be any of those previously described as examples of the cycloolefin resin that may be used as a material of the shaped products [1].

The cycloolefin resin [4] may include an alkoxysilyl group. Introduction of an alkoxysilyl group into the cycloolefin resin [4] can be performed by graft polymerization or end modification. For example, introduction of an alkoxysilyl group to the cycloolefin resin [4] can be performed by impregnation of pellets with an alkoxysilane compound and a peroxide under stirring. The alkoxysilane compound may be trimethoxyvinylsilane, vinyltriethoxysilane, 3-methacryloxypropyltrimethoxysilane, or the like, for example. The inclusion of an alkoxysilyl group in the cycloolefin resin [4] can provide good adhesiveness.

The number-average molecular weight of the cycloolefin resin [4] may be preferably 12,500 or more, and more preferably 13,000 or more. Moreover, the number-average molecular weight of the cycloolefin resin [4] may be preferably 200,000 or less, more preferably 100,000 or less, and even more preferably 80,000 or less. When the number-average molecular weight of the cycloolefin resin [4] is within any of the ranges set forth above, it is possible to obtain good fluidity during melt-shaping and strength after shaping.

The glass-transition temperature of the cycloolefin resin [4] is preferably 100°C or lower, more preferably 90°C or lower, and even more preferably 85°C or lower. Moreover, the glass-transition temperature of the cycloolefin resin [4] is preferably 50°C or higher, more preferably 60°C or higher, and even more preferably 65°C or higher. When the glass-transition temperature of the cycloolefin resin [4] is within any of the ranges set forth above, it is possible to obtain good formability at low temperature and shape retention in the temperature range of use.

The softening agent [5] is a substance that lowers the softening temperature of a resin mixed therewith and is a substance that results in a mixture having a haze of 1.0 or less when mixed with the cycloolefin resin that is used in the joining layer. In a case in which the presently disclosed assembly [3] is to be used as a microchannel chip for analysis or testing of a biological substance such as DNA, RNA, or a protein, the softening agent [5] is preferably a substance to which such biological substances do not adsorb. The softening agent [5] may be an olefin-based low molecular weight substance or hydrogenated product thereof such as a hydrogenated product of liquid paraffin, polyisobutylene, polybutene, poly-4-methylpentene, poly-1-octene, or ethylene-α-olefin copolymer; a conjugated diene-based low molecular weight substance or hydrogenated product thereof such as polyisoprene or polyisoprene-butadiene copolymer; or the like, for example, and is preferably a hydrogenated product of liquid paraffin. The density of the hydrogenated product of liquid paraffin is preferably 0.82 g/cm³ to 0.89 g/cm³. The kinematic viscosity of the hydrogenated product of liquid paraffin is preferably 4.0 mm²/s to 89.0 mm²/s. The SUS viscosity of the hydrogenated product of liquid paraffin is preferably 38 to 420. The hydrogenated product of liquid paraffin may be a commercially available product such as HICALL K-350 (produced by Kaneda Co., Ltd.) or HICALL K-140N (produced by Kaneda Co., Ltd.). Compounding of the softening agent [5] is advantageous in terms that the glass-transition temperature of the joining layer [2] can be lowered and that the shaped products can be joined at a comparatively low temperature, thereby inhibiting deformation caused by heating during joining and facilitating peeling apart of the assembly. Moreover, by using a hydrogenated product of liquid paraffin as the softening agent [5], protein adsorption to the joining layer is suppressed, and low joining layer haze is achieved.

The thickness of the joining layer may, for example, be 100 µm or less, preferably 50 µm or less, and more preferably 30 µm or less. When the joining layer has a thinner thickness, channel deformation during steam sterilization treatment is inhibited in the case of use as a microchannel chip, for example, as a result of the joining layer being a thin film. The thickness of the joining layer should be of a minimum thickness that makes it possible to ensure adhesiveness of a channel substrate and a lid substrate and may, for example, be 0.1 µm or more, preferably 0.5 µm or more, and more preferably 1.0 µm or more.

### (Method for manufacturing assembly)

The presently disclosed assembly [3] can be produced by a method described below (hereinafter, referred to as the "presently disclosed method"), for example.

The presently disclosed method includes joining shaped products [1] via a joining layer [2] through thermal fusion. More specifically, the presently disclosed method includes a step of forming a joining layer [2] on at least one shaped product [1], a step of stacking shaped products [1] with the joining layer [2] in-between to form a temporarily fixed assembly, and a step of heating the temporarily fixed assembly to cause thermal fusion.

The means by which thermal fusion is performed may be an autoclave, a vacuum plate press, a vacuum diaphragm-type laminator, a hot roll press, or the like, for example. The temperature at which thermal fusion is performed is preferably 25°C or higher, and more preferably 35°C or higher. Moreover, the temperature at which thermal fusion is performed is preferably 100°C or lower, and more preferably 60°C or lower. Although it is preferable that before performing thermal fusion, trapped air is released from the temporarily fixed assembly and pressure bonding is performed, a small amount of air bubbles are expelled during processing in the case of a depressurizing mechanism such as a vacuum plate press and diffuse during processing in the case of a pressurizing mechanism such as an autoclave, and thus a problem does not arise so long as a large amount of air is not trapped.

In a case in which the presently disclosed assembly [3] is a microchannel chip, the presently disclosed method may be implemented by an order in which a joining layer [2] is formed over the entire surface of a shaped product [1] and then a channel is subsequently formed on a shaped product [1]. In other words, the presently disclosed method may be implemented through the following steps.
(1) A step of forming a joining layer [2] on at least one shaped product [1]
(2) A step of forming a channel by cutting, photolithography, or thermal imprinting on a shaped product [1] on which a joining layer [2] has been formed or a shaped product [1] on which a joining layer [2] has not been formed
(3) A step of joining a shaped product [1] on which a channel has been formed and another shaped product [1] via a joining layer [2] through thermal fusion

Alternatively, in a case in which the presently disclosed assembly [3] is a microchannel chip, the presently disclosed method may be implemented by an order in which a shaped product [1] having a channel formed thereon is produced and then a joining layer [2] is subsequently formed on a surface of a shaped product [1] at a section of the surface that is not a section corresponding to a channel. In other words, the presently disclosed method may be implemented by the following steps.
(1) A step of forming a shaped product [1] having a channel formed thereon
(2) A step of forming a joining layer [2] on a surface of a shaped product [1] having a channel formed thereon or a shaped product [1] not having a channel formed thereon at a section of the surface that is not a section corresponding to a channel
(3) A step of joining shaped products [1] via a joining layer [2] through thermal fusion with a proviso that a channel is formed on at least one of the shaped products [1] and a joining layer [2] is formed on at least one of the shaped products [1]

In a case in which a shaped product [1] used in the presently disclosed assembly [3] is an organic material other than a cycloolefin resin, such as polycarbonate or acrylic, the presently disclosed method may further include subjecting a surface of that shaped product [1] to one or more treatments selected from the group consisting of plasma irradiation, ultraviolet irradiation, corona discharge, and flame spraying.

### (Peeling apart of assembly)

The presently disclosed assembly [3] may, for example, be peeled apart for recycling. Peeling apart of the assembly [3] can be performed under conditions in which the assembly [3] is heated by a dryer or the like or by leaving the assembly [3] for a specific time in an oven that is heated to a temperature at which the joining layer softens.

### EXAMPLES

The following provides a more specific description of the present disclosure based on examples. However, the present disclosure is not limited to the following examples.

### <Methods for measuring and evaluating physical properties>

Measurements and evaluations of various physical properties were performed according to the following methods.

### (Method for measuring number-average molecular weight Mn)

Number-average molecular weight Mn was determined as a standard polyisoprene-equivalent value through measurement by gel permeation chromatography (GPC) with cyclohexane as an eluent. Standard polyisoprene produced by Tosoh Corporation was used as the standard polyisoprene. In a case in which the sample did not dissolve in cyclohexane, number-average molecular weight Mn was determined as a standard polystyrene-equivalent value through measurement by GPC with tetrahydrofuran (THF) as an eluent. Standard polystyrene produced by Tosoh Corporation was used as the standard polystyrene.

### (Method for measuring glass-transition temperature)

Glass-transition temperature (Tg) was measured using a differential scanning calorimeter (produced by SII NanoTechnology Inc.; product name: DSC6220SII) based on JIS-K7121 under conditions of a heating rate of 10°C/min.

### [1. Production of cycloolefin polymers (COPs)]

### <Production of COP-Tg156>

### (1-1) Production of ring-opened polymer

At room temperature, 200 parts by weight of dehydrated cyclohexane, 0.75 mol% of 1-hexene, 0.15 mol% of diisopropyl ether, and 0.44 mol% of triisobutylaluminum relative to 100 parts by weight, in total, of subsequently described monomers were loaded into a glass reactor that had been internally purged with nitrogen and were mixed. Thereafter, 40 parts by weight of methanotetrahydrofluorene (MTF), 56 parts by weight of tetracyclododecene (TCD), and 4 parts by weight of dicyclopentadiene (DCPD) as monomers and 0.02 mol% of tungsten hexachloride (0.65 weight% toluene solution) were continuously added into the reactor over 2 hours, concurrently to one another, and polymerization was performed while maintaining a temperature of 45°C. Next, 0.2 mol% of isopropyl alcohol was added to the polymerization solution so as to deactivate the polymerization catalyst and stop the polymerization reaction. Note that amounts given in units of "mol%" in the preceding description are each a value for when the total amount of monomers is taken to be 100 mol%. The resultant norbornene-based ring-opened polymer had a number-average molecular weight (Mn) of 1.33 × 10⁴, a weight-average molecular weight (Mw) of 2.8 × 10⁴, and a molecular weight distribution (Mw/Mn) of 2.1. The conversion rate of monomers to polymer was 100%.

### (1-2) Production of norbornene-based cycloolefin polymer (COP-Tg156) by hydrogenation

Next, 300 parts of a reaction solution containing the ring-opened polymer obtained by step (1-1) was transferred to a stirrer-equipped autoclave, 3 parts of nickel catalyst loaded on diatomaceous earth (T8400RL produced by JGC C&C; nickel loading rate: 57%) was added, and a hydrogenation reaction was performed at a hydrogen pressure of 4.5 MPa and a temperature of 160°C for 4 hours.

Once the hydrogenation reaction had ended, the resultant solution was subjected to pressurized filtering (FUNDABAC Filter produced by Ishikawajima-Harima Heavy Industries Co., Ltd.) at a pressure of 0.25 MPa using Radiolite #500 as a filter bed so as to remove the hydrogenation catalyst and thereby obtain a colorless and transparent solution. The obtained solution was poured into a large amount of isopropanol to cause precipitation of a norbornene-based cycloolefin polymer (COP-Tg156) as a hydrogenated ring-opened polymer. The norbornene-based cycloolefin polymer (COP-Tg156) that precipitated was collected by filtration and was subsequently dried by a vacuum dryer (220°C, 1 Torr) for 6 hours to yield the norbornene-based cycloolefin polymer (COP-Tg156). The norbornene-based cycloolefin polymer (COP-Tg156) had a number-average molecular weight (Mn) of 1.52 × 10⁴, a weight-average molecular weight (Mw) of 3.5 × 10⁴, and a molecular weight distribution of 2.3.

The glass-transition temperature Tg of the obtained norbornene-based cycloolefin polymer (COP-Tg156) was 156°C.

The norbornene-based cycloolefin polymer (COP-Tg156) obtained in step (1-2) was loaded into a twin-screw extruder and was shaped into the form of a strand-like shaped product through hot-melt extrusion molding. This shaped product was finely cut using a strand cutter to obtain pellets of a thermoplastic norbornene-based resin that contained the norbornene-based cycloolefin polymer (COP-Tg156).

### <Production of COP-Tg68 (twin-screw kneading reaction)>

A norbornene-based cycloolefin polymer (COP-Tg68) and pellets of a thermoplastic norbornene-based resin containing the COP-Tg68 were obtained in the same way as in production of COP-Tg156 with the exception that 31 parts by weight of tetracyclododecene (TCD), 33 parts by weight of dicyclopentadiene (DCPD), and 36 parts by weight of norbornene (NB) were used as monomers. The COP-Tg68 had a number-average molecular weight (Mn) of 13,000 and a glass-transition temperature Tg of 68°C.

### <Production of silane-modified COP-Tg68>

After weighing out 2 parts by weight of trimethoxyvinylsilane (KBM-1003 produced by Shin-Etsu Silicone) and 0.1 parts by weight of a peroxide (PERHEXA 25B produced by NOF Corporation) relative to 100 parts by weight of the thermoplastic norbornene-based resin containing COP-Tg68, these materials were impregnated to the pellets under stirring inside a Henschel mixer (Super Mixer SMV produced by Kawata Mfg. Co., Ltd.). Externally lubricated COP-Tg68 resin was loaded into a weighing hopper of a twin-screw kneader (TEM-37B produced by Shibaura Machine Co., Ltd.), melt kneading was performed with a screw barrel temperature of 220°C, a screw rotation speed of 150 rpm, and a residence time of 90 seconds, and removed strands were pelletized by a pelletizer (FAN-CUTTER produced by Hoshi Plastic Co., Ltd.) while being cooled in a water tank to thereby produce silane-modified COP-Tg68 (hereinafter, referred to as "Si-COP-Tg68"). The Si-COP-Tg68 had a number-average molecular weight (Mn) of 12,700 and a glass-transition temperature Tg of 65°C.

### <Production of COP-Tg138>

A norbornene-based cycloolefin polymer (COP-Tg138) and pellets of a thermoplastic norbornene-based resin containing the COP-Tg138 were obtained in the same way as in production of COP-Tg156 with the exception that 27 parts by weight of methanotetrahydrofluorene (MTF), 35 parts by weight of tetracyclododecene (TCD), and 38 parts by weight of dicyclopentadiene (DCPD) were used as monomers. The COP-Tg138 had a number-average molecular weight (Mn) of 13,000 and a glass-transition temperature Tg of 138°C.

### [2. Production of base material shaped products]

### <Production of base material shaped product by injection molding>

Pellets of COP-Tg156 resin were dried for 5 hours at Tg - 20°C. Thereafter, the pellets were injection molded by a standard method using an injection molding machine (FANUC ROBOSHOT^{®} α100B (FANUC ROBOSHOT is a registered trademark in Japan, other countries, or both) produced by FANUC Corporation) with a resin temperature of Tg + 150°C, a mold temperature of Tg - 10°C, and a holding pressure of 80 MPa to obtain a base material shaped product (hereinafter, referred to as an "injection-molded shaped product") as a flat plate of 100 mm × 100 mm × 2 mm.

### <Production of base material shaped product by sheet forming>

COP-Tg156 resin was loaded into a hopper of a single-screw extruder (Single-Layer Extruder produced by GSI Creos Corporation) in which a T-die had been installed, was extruded with a barrel temperature of 260°C and a screw rotation speed of 80 rpm, and was wound up while being cooled and fixed by a mirror finished cooling roll so as to obtain a base material shaped product (hereinafter, referred to as a "sheet shaped product") as a sheet of 125 µm in thickness.

### <Base material shaped product having stainless steel as material>

SUS304 (produced by Nippon Kinzoku Co., Ltd.) was used as a stainless steel material. The stainless steel material was cut to a flat plate of 100 mm × 100 mm × 2 mm so as to obtain a base material shaped product.

### <Base material shaped product having corona discharge-treated polycarbonate resin as material>

Corona discharge-treated polycarbonate resin obtained by performing corona discharge treatment with respect to one side of Panlite pc-2151 #125 (produced by Teijin Limited) was used as corona discharge-treated polycarbonate resin. The corona discharge-treated polycarbonate resin was cut to a flat plate of 100 mm × 100 mm × 2 mm so as to obtain a base material shaped product.

### [3. Formation of joining layer on release film]

A specific number of parts by weight of each softening agent relative to 100 parts by weight of resin was weighed out, and the total amount was taken to be the solid content. A cyclohexane solution having a solid content concentration of 20 parts by weight relative to 100 parts by weight of solution was then produced by hermetically sealing 80 parts by weight of cyclohexane (special grade; produced by Wako Pure Chemical Industries, Ltd.) in a glass hermetically sealed vessel (Pyrex^{®} (Pyrex is a registered trademark in Japan, other countries, or both) media bottle produced by Corning Incorporated) with 20 parts by weight of this solid content and performing shaking thereof under heating at 60°C to cause dissolution.

The obtained solution was cast onto a silicone surface side of silicone-coated PET 50µ (Separator SP-PET produced by Mitsui Chemicals Tohcello, Inc.) that was fixed on a glass plate, and an applicator (Doctor Blade Film Applicator produced by Allgood) having a gap of 300 µm was used to perform wet coating. The coated film was dried at room temperature for approximately 10 minutes and was then thermally dried in an 80°C oven for 1 hour so as to obtain a joining layer-coated film having a joining layer of 30 µm in thickness formed on the silicone-coated PET 50µ (release film).

### [4. Mating of base material shaped products via joining layer]

A sheet shaped product was cut to dimensions of 125 µm (thickness) × 26 mm × 76 mm and a joining layer-coated film was cut to dimensions of 80 µm (thickness) × 26 mm × 76 mm with respect to a shaped plate of 3 mm (thickness) × 26 mm × 76 mm. The joining layer-coated film was stacked and aligned on the shaped plate that had been cut with the joining layer side thereof facing toward the shaped plate. After this alignment, a rubber roll was pressed thereagainst a number of times so as to release trapped air and perform pressure bonding. After pressure bonding, just silicone-coated PET (release film) was peeled off, thereby producing a temporarily fixed assembly A in which the joining layer was provided on the shaped plate. The sheet shaped product of 125 µm in thickness was stacked on the temporarily fixed assembly A such that misalignment did not occur, and a rubber roll was pressed thereagainst a number of times so as to release air bubbles and produce a temporarily fixed assembly B. Note that since a small amount of air bubbles diffuse during processing in an autoclave, a problem does not arise so long as a large amount of air is not trapped.

The temporarily fixed assembly B was loaded into a retort pouch bag (produced by Meiwa Pax Co., Ltd.) and was then subjected to degassed packaging in a vacuum packaging machine (TECNOVAC T1000 produced by Nippon Hoso-Kikai Co., Ltd.). The degassed package was then loaded into an autoclave vessel (DANDELION DL-2010 produced by Hanyuda Co. Jp.) and was subjected to joining and defoaming treatment at 30°C and 0.5 MPa with a heating and pressing time of 15 minutes to obtain an assembly.

### [5. Evaluation]

### <Adhesive strength>

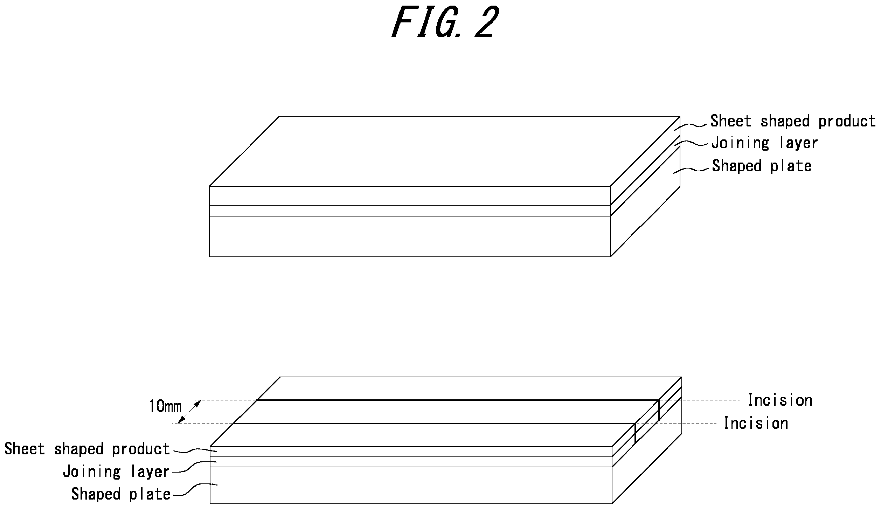

Adhesive strength was measured in accordance with JIS K 6854-2 (180° peeling). Incisions with a width of 10 mm were made at a sheet shaped product side of an assembly such as to pass through the sheet shaped product and a joining layer as illustrated in FIG. 2. A section of 10 mm in width where the incisions had been made was peeled from the edge to enable chuck fixing. The injection-molded shaped product side was fixed to a lower chuck and the sheet shaped product side was fixed to an upper chuck of an oven-equipped universal tester (Autograph AGS-X10kN produced by Shimadzu Corporation), and the assembly was held therein for 5 minutes from the stage at which the temperature inside the oven, which was set to an oven temperature of 38°C, stabilized at 38°C ± 1°C. After this holding, 180° peeling was performed with a peeling width of 10 mm and a peeling rate of 100 mm/min in order to determine the peel strength, which was taken to be the adhesive strength. Measurement at 80°C was performed in the same manner by only changing the oven temperature while keeping other operations the same.

### <Haze>

A joining layer-coated film was cut to the same dimensions as microslide glass (S3132 produced by Matsunami Glass Ind., Ltd.) of 28 mm × 48 mm and was stacked and aligned on the microslide glass with an adhesion surface thereof facing toward the microslide glass. After this alignment, a rubber roll was pressed thereagainst a number of times so as to release trapped air and perform pressure bonding. After pressure bonding, just silicone-coated PET was peeled off, and then microslide glass (S3132 produced by Matsunami Glass Ind., Ltd.) of the same dimensions was stacked and aligned on the surface of the joining layer that had been peeled. After this alignment, a rubber roll was used to perform pressure bonding while releasing trapped air to thereby obtain a temporarily fixed assembly C.

The temporarily fixed assembly C was loaded into a retort pouch bag (produced by Meiwa Pax Co., Ltd.) and was then subjected to degassed packaging in a vacuum packaging machine (TECHNOVAC T1000 produced by Nippon Hoso-Kikai Co., Ltd.). The degassed package was then loaded into an autoclave vessel (DANDELION DL-2010 produced by Hanyuda Co. Jp.) and was subjected to joining and defoaming treatment at 30°C and 0.5 MPa with a heating and pressing time of 15 minutes to obtain an assembly for haze measurement.

The obtained assembly for haze measurement was then subjected to haze measurement using a haze meter (NDH-7000SP produced by Nippon Denshoku Industries Co., Ltd.). Note that the microslide glass by itself had a haze measurement value of less than 0.1, and a value obtained by subtracting this value as a blank was taken to be the haze.

### <Adsorbed amount of BSA>

Fetal bovine serum albumin (BSA) was used to measure adsorption to a joining layer by the following method (micro BCA method) as a protein adsorption test.

### (1. Preparation of adhesive solution)

A specific number of parts by weight of each softening agent relative to 100 parts by weight of resin was weighed out, and the total amount was taken to be the solid content. A cyclohexane solution having a solid content concentration of 20 parts by weight was then produced by hermetically sealing 80 parts by weight of cyclohexane (special grade; produced by Wako Pure Chemical Industries, Ltd.) in a glass hermetically sealed vessel (Pyrex^{®} media bottle produced by Corning Incorporated) with 20 parts by weight of this solid content and performing shaking thereof under heating at 60°C to cause dissolution.

### (2. Production of adsorbent sample)

In a 35 mm petri dish (Nunc Petri Dish 150460 produced by Thermo Fisher Scientific), 1 mL of the cyclohexane solution produced in section 1 described above was added per 35 mm dish, was caused to spread across the entire bottom surface of the dish, was dried at normal temperature for approximately 10 minutes, and was then thermally dried in an 80°C oven for 1 hour so as to prepare an adhesive-coated dish.

### (3. Protein adsorption treatment and extraction)

After loading 2.5 mL of BSA solution that had been adjusted to 1 mg/mL with phosphate buffer into the adhesive-coated dish, 2 hours of resting was performed in a 37°C incubator to allow adsorption of BSA. The sample was then removed from the incubator, the BSA solution was withdrawn, and washing was performed while rinsing with PBS solution. Next, 2.5 mL of 1 wt% sodium dodecyl sulfate (SDS) solution was loaded into the dish, and extraction was performed through 5 minutes of treatment by an ultrasonic washer.

### (4. Quantification of protein)

A 96-well plate was loaded with 150 µL each of BSA solutions from 20 mg/mL to blank (i.e., only PBS) for calibration curve preparation and with 150 µm of the extracted solution obtained in section 3 described above for measurement. Next, using a micro BCA kit (Micro BCA Assay Kit produced by Thermo Fisher Scientific), kit reagents were loaded into the 96-well plate in an amount of 150 µL each and were slowly agitated for approximately 30 seconds using a plate shaker. The 96-well plate was sealed in order to prevent evaporation and was left in a 37°C incubator for 2 hours to allow reaction. After this reaction, light absorbance at 562 mm was measured by a plate reader and was converted to an adsorbed amount of protein using a calibration curve.

### <Warping>

A sheet shaped product was prepared with dimensions of 125 µm (thickness) × 15 mm × 100 mm and a joining layer-coated film was prepared with dimensions of 80 µm (thickness) × 15 mm × 76 mm with respect to an injection-molded shaped product of 3 mm (thickness) × 15 mm × 100 mm in the same manner as in "4. Mating of base material shaped products via joining layer".

Measurement of warping was performed by, after mating had been performed in an autoclave, placing the removed assembly on a horizontal plate such as a glass plate and measuring lifting up of both edges of the assembly using a caliper so as to determine the warping.

### [7. Results]

Production conditions and results of evaluations are shown in Table 1.

**Table 1-1**

| | Requirement | | Scope | Example 1 | Example 2 | Example 3 | Example 4 | Example 5 | Example 6 | Example 7 | Example 8 | Example 9 | Example 10 |
|---|---|---|---|---|---|---|---|---|---|---|---|---|---|
| | Shaped products | Lid material (extruded sheet; 100 µm) | Cycloolefin resin | COP- Tg156 | COP- Tg156 | COP- Tg156 | COP- Tg156 | COP-Tgl56 | COP- Tg156 | COP- Tg156 | COP- Tg156 | COP-Tg156 | COP-Tg156 |
| | | | Glass-transition temperature [°C] | 156 | 156 | 156 | 156 | 156 | 156 | 156 | 156 | 156 | 156 |
| | | Base material (injection-molded shaped plate; 3 mm thickness) | Cycloolefin resin | COP-Tg156 | COP-Tg156 | COP-Tg156 | COP- Tg156 | COP-Tg156 | COP-Tg156 | SUS304 produced by Nippon Kinzoku | SUS304 | Polycarbonate (corona treated) Panlite pc-2151 #125 | Polycarbonate (corona treated) Panlite pc-2151 #125 |
| | | | Glass-transition temperature [°C] | 156 | 156 | 156 | 156 | 156 | 156 | | | 150 | 150 |
| Production conditions | Joining agent | Cycloolefin resin | | COP-Tg68 | COP-Tg68 | COP-Tg68 | Si-COP-Tg68 | Si-COP-Tg68 | Si-COP-Tg68 | COP-Tg68 | Si-COP-Tg68 | COP-Tg68 | Si-COP-Tg68 |
| | | Glass-transition temperature [°C] | | 68 | 68 | 68 | 65 | 65 | 65 | 68 | 65 | 68 | 65 |
| | | Number-average molecular weight (Mn) | | 13000 | 13000 | 13000 | 12700 | 12700 | 12700 | 13000 | 12700 | 13000 | 12700 |
| | | Parts by weight of softening agent per 100 parts by weight of resin | | 11 | 33 | 67 | 11 | 33 | 67 | 33 | 33 | 33 | 33 |
| | | Softening agent product name | | Hydrogenated paraffin HICALLK-350 (produced by Kaneda) | Hydrogenated paraffin HICALLK-350 (produced by Kaneda) | Hydrogenated paraffin HICALLK-350 (produced by Kaneda) | Hydrogenated paraffin HICALLK-350 (produced by Kaneda) | Hydrogenated paraffin HICALL K-350 (produced by Kaneda) | Hydrogenated paraffin HICALLK-350 (produced by Kaneda) | Hydrogenated paraffin HICALL K-350 (produced by Kaneda) | Hydrogenated paraffin HICALLK-350 (produced by Kaneda) | Hydrogenated paraffin HICALLK-350 (produced by Kaneda) | Hydrogenated paraffin HICALL K-350 (produced by Kaneda) |
| | Mating conditions | Autoclave | | Joining sheet 30 µm thickness 30°C, 0.1 Pa, 15 mins, | Joining sheet 30 pmthickness 30°C, 0.1 Pa, 15 mins | Joining sheet 30 µm thickness 30°C, 0.1 Pa, 15 mins | Joining sheet 30 µm thickness 30°C, 0,1 Pa, 15 mins | Joining sheet 30 µm thickness 30°C, 0.1 Pa, 15 mins | Joining sheet 30 pmthickness 30°C, 0.1 Pa, 15 mins | Joining sheet 30 µm thickness 30°C, 0.1 Pa, 15 mins | Joining sheet 30 µm thickness 30°C, 0.1 Pa, 15 mins | Joining sheet 30 µm thickness 30°C, 0.1 Pa, 15 mins | Joining sheet 30 µm thickness 30°C, 0.1 Pa, 15 mins |
| Evaluations | Dimensional stability (warping of assembly) [mm] | | | <0.1 | <0.1 | <0.1 | <0.1 | <0.1 | <0.1 | <0.1 | <0.1 | <0.1 | <0.1 |
| | Transparency of joining layer (haze) | | | 0.2 | 0.2 | 0.2 | 0.2 | 0.2 | 0.2 | 0.2 | 0.2 | 0.2 | 0.2 |
| | Adhesive strength [N/10 mm] (38°C peeling) 2 N/10 mm or more is good | | | 4.8 | 5.5 | 5.8 | 6.9 | 8.2 | 7.7 | 2.8 | 9.1 | 4.9 | 6.5 |
| | Adhesive strength [N/1 0 mm] (80°C peeling) Less than 2 N/10 mm is good | | | 0.8 | 0.5 | 0.2 | 0.8 | 0.6 | 0.2 | 0.5 | 0.8 | 0.5 | 0.6 |
| | Adsorbed amount of protein (BSA) [µg/cm2] 0.4 or less is good with low adsorption | | | 0.25 | 0.25 | 0.25 | 0.25 | 0.25 | 0.25 | 0.25 | 0.25 | 0.25 | 0.25 |

| | | | | | | | | | | | | | |
|---|---|---|---|---|---|---|---|---|---|---|---|---|---|
| Adsorbed amount of protein (BSA: Bovine Serum Albumin) measured at surface where shaped material is coated with joining agent | | | | | | | | | | | | | |

**Table 1-2**

| | Requirement | | Scope | Comparative Example 1 | Comparative Example 2 | Comparative Example 3 | Comparative Example 4 | Comparative Example 5 | Comparative Example 6 |
|---|---|---|---|---|---|---|---|---|---|
| Production conditions | Shaped products | Lid material (extruded sheet; 100 µm) | Cycloolefin resin | COP-Tg156 | COP-Tg156 | COP-Tg156 | COP-Tg156 | COP-Tg156 | COP-Tg156 |
| | | | Glass-transition temperature [°C] | 156 | 156 | 156 | 156 | 156 | 156 |
| | | Base material (injection-molded shaped plate; 3 mm thickness) | Cycloolefin resin | COP-Tg156 | COP-Tg156 | COP-Tg156 | COP-Tg156 | COP-Tg156 | COP-Tg156 |
| | | | Glass-transition temperature [°C] | 156 | 156 | 156 | 156 | 156 | 156 |
| | Joining agent | Cycloolefin resin | | COP-Tg68 | COP-Tg68 | Styrene-butadiene block Asaflex 830 (produced by Asahi Kasei) | COP-Tg68 | COP-Tg68 | COP-Tg68 |
| | | Glass-transition temperature [°C] | | 68 | 68 | 95 | 68 | 68 | 68 |
| | | Number-average molecular weight (Mn) | | 13000 | 13000 | 100000 | 13000 | 13000 | 13000 |
| | | Parts by weight of softening agent per 100 parts by weight of resin | | 33 | 33 | 33 | 0 | 5 | 150 |
| | | Softening agent product name | | Hydrogenated polyisobutene Polybutene 30SH (produced by NOF) | Hydrogenated polybutadiene BI-3000 (produced by Nippon Soda) | Hydrogenated paraffin HICALL K-350 (produced by Kaneda) | - | Hydrogenated paraffin HICALL K-350 (produced by Kaneda) | Hydrogenated paraffin HICALLK-350 (produced by Kaneda) |
| | Mating conditions | Autoclave | | Joining sheet 30 µm thickness 30°C, 0.1 Pa, 15 mins | Joining sheet 30 µm thickness 30°C, 0.1 Pa, 15 mins | Joining sheet 30 µm thickness 30°C, 0.1 Pa, 15 mins | Joining sheet 30 µm thickness 30°C, 0.1 Pa, 15 mins | Joining sheet 30 µm thickness 30°C, 0.1 Pa, 15 mins | Joining sheet 30 µm thickness 30°C, 0.1 Pa, 15 mins |
| Evaluations | Dimensional stability (warping of assembly) [mm] | | | <0.1 | <0.1 | <0.1 | <0.1 | <0.1 | <0.1 |
| | Transparency of joining layer (haze) | | | 1.9 | 2.2 | 0.2 | - | 0.2 | 0.2 |
| | Adhesive strength [N/10 mm] (38°C peeling) 2 N/10 mm or more is good | | | 3.9 | 3.9 | 1.8 | Not adhered | 0.5 | 1.3 |
| | Adhesive strength [N/10 mm] (80°C peeling) Less than 2 N/10 mm is good | | | 0.5 | 0.6 | 0.2 | Not adhered | 0.2 | 0.2 |
| | Adsorbed amount of protein (BSA) [µg/cm2] 0.4 or less is good with low adsorption | | | 0.33 | 0.35 | 0.48 | - | 0.25 | 0.25 |

| | | | | | | | | | |
|---|---|---|---|---|---|---|---|---|---|
| Adsorbed amount of protein (BSA: Bovine Scrum Albumin) measured at surface where shaped material is coated with joining agent | | | | | | | | | |

### INDUSTRIAL APPLICABILITY

According to the present disclosure, it is possible to provide an assembly and method for manufacturing the same with which deformation caused by heating during joining is inhibited, peeling apart of the assembly is facilitated, protein adsorption is suppressed, and low haze is achieved.

## Claims

1. An assembly [3] comprising at least one or more types of shaped products [1] that are joined via a joining layer [2] having a material constitution different from the shaped products, wherein
the joining layer [2] comprises: 100 parts by weight of a cycloolefin resin [4]; and a softening agent [5],
content of the softening agent [5] in the joining layer [2] is 6 parts by weight to 99 parts by weight of the softening agent [5] relative to 100 parts by weight of the cycloolefin resin [4], and
the joining layer [2] has a haze of 1.0 or less.

2. The assembly [3] according to claim 1, wherein the cycloolefin resin [4] has a number-average molecular weight of 12,500 or more.

3. The assembly [3] according to claim 1 or 2, wherein the cycloolefin resin [4] has a glass-transition temperature of 100°C or lower.

4. The assembly [3] according to any one of claims 1 to 3, wherein the softening agent [5] is formed of a hydrogenated product of liquid paraffin.

5. The assembly [3] according to any one of claims 1 to 4, wherein the cycloolefin resin [4] has an alkoxysilyl group.

6. The assembly [3] according to any one of claims 1 to 5, wherein the shaped products [1] are an organic material, and a surface of the shaped products [1] is subjected to one or more treatments selected from the group consisting of plasma irradiation, ultraviolet irradiation, corona discharge, and flame spraying.
